# EUROPEAN PATENT APPLICATION

(11) **EP 2 241 614 A1**
(43) Date of publication of application: **20.10.2010**
(21) Application number: 09700562.3
(22) Date of filing: 09.01.2009
(51) Int. Cl.: C12M 3/00, C12M 1/14, C12M 1/40, C12Q 1/00, C12N 5/06

(54) **INSTRUMENT AND METHOD FOR TREATING MINOR AMOUNT OF BIOLOGICAL MATERIAL**

(30) Priority: 10.01.2008 JP 2008003672
(71) Applicant: The University of Tokyo, Bunkyo-Ku Tokyo 113-8654 (JP)
(72) Inventor: TAKEUCHI, Shoji, Tokyo 113-8654 (JP); ONOE, Hiroaki, Tokyo 113-8654 (JP)
(74) Representative: MERH-IP Matias Erny Reichl Hoffmann
(86) International application number: PCT/JP2009/050255
(87) International publication number: WO 2009/088088

(57) **Abstract**

Provided is a technique for handling a minor amount of a biological material with the purpose of observing selectively the interaction between single cells by controlling cell number and two- or three-dimensional configurations of each of the different cell types. The present invention provides an instrument and a method for handling a minor amount of a biological material. The instrument of the present invention for handling a minor amount of a biological material comprises a construct and a base, wherein: the base holds the construct in a releasable manner; the construct is held on a surface of the base in such a manner that a part of the construct is exposed externally on the outer face of the instrument; and at least a part of a surface of the constructs is exposed externally on the outer face of the instrument being fabricated so as to allow the adhesion of the minor amount of the biological material thereto.

## Description

### Technical Field

The present invention relates to an instrument and a method for treating, or handling, a minor amount of a biological material; a method for analyzing interaction between biological materials in minor amounts; and more specifically to an instrument and a method for handling single cells adhered to a substrate, and a method for analyzing the individual interaction between single cells.

### Background Art

Development of a technique for individually handling a minor amount of a biological material, such as single cells adhered to a substrate, is an important challenge in order to understand a biological phenomenon by single molecule observation and to devise engineering applications. Non-Patent Document 1 reports a prior art approach, wherein the cell adhesiveness of a substrate in a culture vessel is sequentially changed using a parylene stencil, which has been fabricated with a MEMS technique, thereby allowing mixed culture of cells of different cell types adhered to the substrate in a specific pattern. Non-Patent Document 2 reports another approach, wherein a plurality of microfabricated constructs with comb fingers are provided, and after adhering a different type of cells to the surface of each construct and by fitting the comb fingers of both constructs together, mixed culture is achieved in which the cells of the plurality of cell types are arranged at a given interval.

### Prior-Art Documents

Non-Patent Document 1: Wright, D. et al., Lab on a Chip, 7: 1272-1279 (2007)
Non-Patent Document 2: Hui, E. E. and Bhatia, S. N., Proc. Natl. Acad. Sci. U.S.A., 104: 5722-5726 (2007)

### Disclosure of the Invention

### (Problems to be solved by the Present Invention)

However, according to these prior arts, interaction occurs between cell populations of different cell types. Therefore, in a strict sense, the interaction between cells of the same cell type and the interaction between cells of different cell types coexist, which makes it impossible to observe selectively the interaction between cells of different cell types. In view of the above problem, there is a need for developing a technique for handling a minor amount of a biological material with the purpose of observing selectively the interaction between single cells by controlling cell number and two- or three-dimensional configurations of each of the different cell types.

### (Means for Solving the Problem)

The present invention provides an instrument for treating or handling a minor amount of a biological material. The instrument of the present invention for handling a minor amount of a biological material includes constructs and a base holding the constructs in a releasable manner, the constructs being held on the surface of the base in such a manner that a part of the constructs is exposed externally on the outer face of the instrument, and at least a part of the surface of the constructs exposed externally on the outer face of the instrument being fabricated so as to allow the adhesion of the minor amount of the biological material thereto.

In the instrument of the present invention, the base is made of adhesive and elastic material. The construct is held on the surface of the elastic base by adhesion in such a manner that a part of the construct is exposed externally on the outer face of the instrument and the construct is released from the elastic base upon application of stress on the elastic base.

In the instrument of the present invention, the minor amount of the biological material may be a single cell for a single construct.

In the instrument of the present invention, the biological material may be an animal cell, and the surface of the construct exposed externally on the outer face of the instrument may have a dimension allowing the extension of only a single animal cell.

In the instrument of the present invention, the minor amount of the biological material may be a single animal cell for two or more constructs, and the surface of a single construct exposed externally on the outer face of the instrument may be smaller than the dimension of the single extended animal cell, and the single animal cell draws the two or more constructs to form a three-dimensional cell scaffold.

In the instrument of the present invention, the construct is optically transparent.

In the instrument of the present invention, the construct may have a self-assembling function.

In the instrument of the present invention, a construct may be provided with a concave surface and/or a convex surface and the self-assembling function may be achieved by complementary engagement between the concave surface of one construct and the convex surface of another construct.

In the instrument of the present invention, a construct may be provided with a hydrophilic surface and/or a hydrophobic surface and the self-assembling function is achieved by a contact between the hydrophilic surface of one construct and the hydrophilic surface of another construct, and/or, a contact between the hydrophobic surfaces of one construct and the hydrophobic surfaces of another construct.

The present invention provides a kit for analyzing a minor amount of a biological material. The kit of the present invention comprises the biological material and the instrument of the present invention for handling a minor amount of a biological material.

In the kit of the present invention, the minor amount of the biological material may be adhered to the instrument.

In the kit of the present invention, the biological material may be a cell.

The present invention provides a method for handling a minor amount of a biological material. The method of the present invention for handling a minor amount of a biological material is comprised of: a step of holding a construct on a surface of a base in a releasable manner, the construct being integrally molded with a substrate and a support which connects the construct to the substrate; a step of disposing liquid on the construct on the base, the liquid suspending a biological material, so that the minor amount of the biological material is adhered on at least a part of a surface of the construct; a step of releasing the construct, to which the biological material is adhered, from the base; and a step of moving the construct to which the biological material is adhered.

In the method of the present invention for handling a minor amount of a biological material, the step of holding the constructs on the surface of the base in a releasable manner may be a step of attaching the construct on the surface of the base by pressing the construct to the surface of the base; and, following the step of attaching the construct, the method is further comprised of: a step of breaking the support by the force of pressing the construct; separating the construct from the substrate; and holding the construct on the surface of the base.

In the method of the present invention for handling a minor amount of a biological material, the step of attaching the construct to the surface of the base and the step of separating the construct from the substrate may be independently carried out for each of a plurality of constructs; and the method may further be comprised of a step of pressing a base holding one of the plurality of constructs with a base holding another of the plurality of constructs, thereby forming a three-dimensional construct in which one of the plurality of constructs is adhered to the surface of another of the plurality of constructs.

In the method of the present invention for handling a minor amount of a biological material, the base may be adhesive and elastic; the construct may be integrally molded with a substrate and a support which connects the construct to the substrate; the step of attaching the construct on the surface of the base may be a step of pressing the adhesive and elastic base to the construct, thereby attaching the constructs to the surface of the elastic base using the elastic force generated in the elastic base; the step of separating the constructs from the substrate may be a step of breaking the supports by the force of pressing the construct, and holding the constructs on the surface of the elastic base by adhesion thereby separating the construct from the substrate; and the step of releasing the construct, to which the biological material is adhered, from the base may be a step of deforming the elastic base under stress thereby releasing the construct, to which the biological material is adhered, from the elastic base.

The method of the present invention for handling a minor amount of a biological material may be comprised of, preceding the step of holding, a step of intensifying the adhesion between the biological material and the construct.

In the method of the present invention for handling a minor amount of a biological material, the minor amount of the biological material may be one cell for one construct.

In the method of the present invention for handling a minor amount of a biological material, the minor amount of the biological material may be a single cell for two or more constructs.

In the method of the present invention for handling a minor amount of a biological material, the construct may have a self-assembling function.

The present invention provides a method for analyzing interaction between minor amounts of biological materials. The method of the present invention for analyzing the interaction between minor amounts of a biological material is comprised of: a step of preparing two or more instruments of the present invention, wherein the construct comprising the instruments has been designed such that a construct derived from one of the instruments self-assemble with a construct derived from another of the instruments, but a construct derived from one of the instruments does not self-assemble with a construct derived from the same instrument; a step of adhering a minor amount of a biological material to the surface of the construct exposed externally on the outer face of the two or more instruments; a step of releasing the constructs, to which the minor amount of the biological material is adhered, from the base of each instruments; a step of allowing self-assembling of the constructs, to which the minor amount of the biological material is adhered, in a mixed state, thereby forming an aggregate composed of the constructs derived from different instruments; a step of allowing reaction to cause interaction between the minor amounts of the biological material adhered to the surface of the constructs forming the aggregate; and a step of analyzing the interaction between the minor amounts of the biological material for the individual aggregate.

In the method of the present invention for analyzing the interaction between minor amounts of a biological material, the type of the biological materials adhered to the construct may be different among the instruments.

The present invention provides an instrument for handling a minor amount of a biological material. The instrument of the present invention consists of a construct and a base, wherein: the base holds the construct in a solvent; at least a part of the surface of the constructs is treated to allow the minor amount of the biological material to adhere thereto; and the construct is held on the base by a specific interaction between the part of the surface of the base and another part of the surface of the constructs.

In the instrument of the present invention, the construct may be made of polyparaxylylene, the solvent may be an aqueous solution, and treatment for allowing the minor amount of the biological material to adhere may be O₂ plasma exposure.

In the instrument of the present invention, the specific interaction may be hydrophobic.

In the instrument of the present invention, another part of the surface of the construct may be treated to become hydrophobic.

In the instrument of the present invention, a part of the surface of the base may be treated to become hydrophobic, and oil droplets may bond thereto.

In the instrument of the present invention, the specific interaction may be electromagnetic.

In the instrument of the present invention, the electromagnetic interaction may be interaction between an electric field and an electric dipole moment induced by the electric field.

In the instrument of the present invention, the specific interaction may be between a biomolecule and a specific binding partner for the biomolecule.

In the instrument of the present invention, the specific interaction may be between a saccharide and a lectin.

In the instrument of the present invention, the specific interaction may be between an antigen and an antibody.

In the instrument of the present invention, the specific interaction may be by complementary pairing of nucleic acids.

In the instrument of the present invention, the specific interaction may be between a metal ion and a chelating agent specific to the metal ion.

The present invention provides an instrument for handling a minor amount of a biological material. The instrument of the present invention is comprised of at least two constructs and a base holding the constructs in a releasable manner, the constructs being held on the surface of the base in such a manner that a part of the constructs is exposed externally on the outer face of the instrument, and at least a part of the surface of the constructs exposed externally on the outer face of the instrument being fabricated so as to allow the adhesion of a minor amount of a biological material thereto.

In the instrument of the present invention, the minor amount of the biological material may be one or more cells, and at least one cell may adhere to the surface of the at least two constructs, thereby drawing the at least two constructs to form a three-dimensional cell scaffold.

In the instrument of the present invention, the at least two constructs may be arranged in such a manner that the three-dimensional cell scaffold forms a convex polyhedron or a part thereof.

The present invention provides a method for forming an aggregate consisting of a cell(s) and a construct(s). The method of the present invention for forming an aggregate is comprised of: a step of preparing a cell and the instrument of the present invention for handling a minor amount of a biological material; a step of inoculating and culturing the cell in such a manner that at least one of the cells adheres to the surface of at least two constructs of the instrument; and a step of allowing the cells on culture to draw the at least two constructs, such that the surface of the constructs encloses the cell to form a three-dimensional cell scaffold, wherein in the three-dimensional cell scaffold, the surface of the constructs is a convex polyhedron or a part thereof.

In the method for forming an aggregate, the construct may include a sensor for a physical property.

The present invention provides a method for measuring a physical property of a cell. The method of the present invention for measuring the physical properties of cells is comprised of: a step of preparing an aggregate of a cell(s) and a construct(s) formed by the method of the present invention for forming an aggregates composed of a cell(s) and a construct(s); and a step of measuring the physical property of the cell using the sensor for the physical property included in at least one of the construct.

In the method of the present invention for measuring the physical properties of a cell, the physical property of the cell may be selected from the group consisting of elasticity, hardness, electric potential, and membrane transport.

### Brief Description of the Drawings

Fig. 1 includes micrographs showing NIH3T3 cells (A) and HepG2 cells (B) adhered to and extended on a substrate.
Fig. 2 includes schematic views showing the instrument and method of the present invention.
Fig. 3 includes an optical micrograph (A), a scanning electron micrograph (B), and a cross section schematic view (C) of the constructs of the present invention immediately before the step of attaching to the surface of the base.
Fig. 4 includes a micrograph (A) and a cross section schematic view (B) showing that the 3T3 cells are selectively adhered not to the base but to the constructs held on the base.
Fig. 5 includes a schematic view (A) showing the release of a construct to which a cell is adhered from a base using a glass tube, and a schematic view (B) showing the movement of the construct by a liquid flow from the glass tube.
Fig. 6 includes sequential photographs showing the movement of a construct to which a cell is adhered by a liquid flow from a glass tube.
Fig. 7 includes a schematic view (A) and micrographs (B), (C), and (D) showing concurrent treatment of cells of different cell types using constructs of different shapes.
Fig. 8 includes schematic views showing the method for producing the instrument of the present invention for handling a minor amount of a biological material.
Fig. 9 includes micrographs showing the experimental results in the examples including the formation of aggregates composed of cells and constructs, using the instrument of the present invention for handling a minor amount a biological material.
Fig. 10 shows another example of the instrument of the present invention for handling a minor amount of a biological material.

### Best Mode for Carrying Out the Invention

The biological material herein refers to any material derived from an organism, and includes, but not limited to, an individual organism, an organ, a tissue and a cell; a cellular organelle such as a mitochondrion, chloroplast, chromosome, mitotic apparatus, lysosome, and Golgi apparatus; a molecular assembly such as a virus, DNA replication enzyme complex, transfer/RNA processing complex, ribosome, ion channel, and cell surface receptor complex; a biopolymer such as nucleic acid, protein, and carbohydrate; and all compounds which interact with other biological material such as hormones, second messenger, enzyme substrate, and metabolic intermediate. Preferred biological material is a cell, especially, a cell of a multicellular organism which interacts with a cell of the other cell type while adhered to a solid substrate.

In the present invention, "the minor amount" of a biological material refers to one cell, one molecule of a biopolymer or compound, or a minimum unit of molecular aggregates for at least one construct. Alternatively, on the precondition that the position of one cell, one molecule of a biopolymer or compound, or a minimum unit of molecular aggregates can be controlled on the surface of the constructs, "the minor amount" of a biological material may refer to a plurality of cells, a plurality of molecules of a biopolymer or compound, or a plurality of minimum units molecular aggregates for at least one construct.

In the present invention, the adhesion of a biological material refers to the adhesion between the surface of the constructs, which are solid substrates, and the biological material. When the biological material is a cell organelle, molecular aggregate, biopolymer, or compound, the adhesion refers to immobilization of the biological material on the constructs through any covalent and/or noncovalent bonds including adsorption by static charges. When the biological material is a cell, the adhesion includes non-physiological phenomenon such as immobilization of the biological material on the constructs through any covalent and/or noncovalent bonds, and cellular physiological phenomenon such as cell-substrate adhesion. When the number of cell is two or more, the adhesion includes intercellular adhesion.

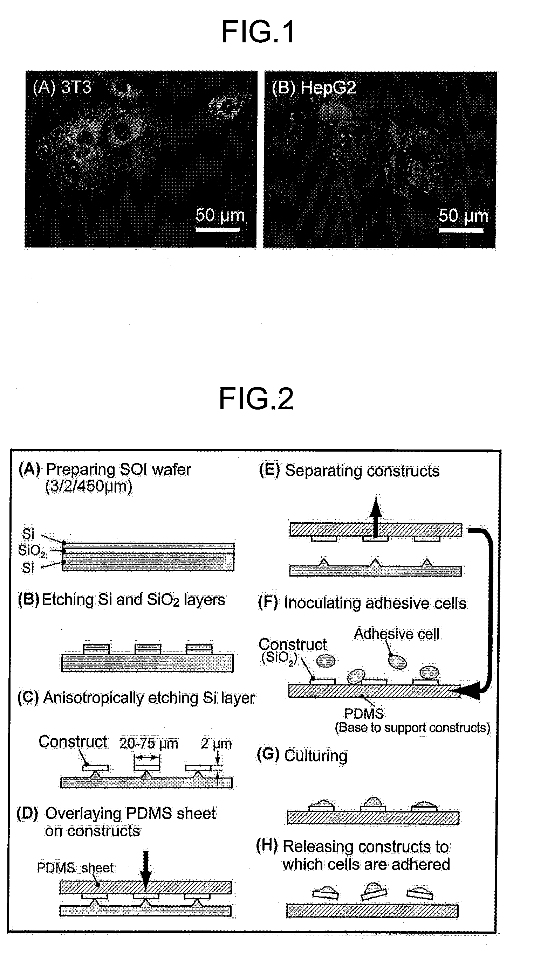

In the present invention, the treatment for allowing adhesion of a minor amount of a biological material refers to the control of the number of the biological material adhered to each construct. Furthermore, the position of the biological material on the surface of the construct may be controlled. Specific procedure of the treatment differs depending on whether the biological material is a cell, cell organelle, molecular aggregate, biopolymer, and/or compound. When the biological material is a cell, the number of cell adhered to the surface of each construct may be controlled by designing the surface dimension of the construct on the basis of, for example, the dimension of the substrate occupied by one extended cell. Furthermore, the surface shape of the construct or adhesiveness between the surface and cell may be changed thereby allowing the cell to adhere only to the specific area on the surface of the construct. The situation wherein one cell is adhered to two or more constructs is achieved under conditions that, for example, the surface dimension of each construct is smaller than the dimension of the substrate occupied by one extended cell, and one cell adheres to the surface of two or more constructs, which are arranged adjacent to each other, when the cell is brought into contact with the surface of the instrument of the present invention. The substrate dimension occupied by one extended cell differs depending on the cell type, adhesiveness between the substrate and cell, and cell cycle, and is, for example, the diameter occupied by one mouse NIH 3T3 cell or human liver cancer HepG2 cell in the resting stage is about 50 to 75 µm (Fig. 1). When the biological material is a molecular aggregate, biopolymer, or compound, the number and position of the biological material adhered to the construct may be controlled using a method known as a so-called monad manipulation technique.

In the construct of the present invention, a plurality of constructs may be adhered to one cell aggregation composed of a plurality of cells. For example, one or more cells adhered to adjacent constructs on a base may grow into a cell aggregation adhered to the plurality of constructs. Alternatively, cells are adhered to self-assembling constructs, and then the constructs are released from the base, and allowed to achieve self-assembling, or the cells are adhered to aggregates of the self-assembling constructs.

In the present invention, the treatment for intensifying the adhesion between a biological material and constructs refers to the coating of the surface of the constructs with a substance known to contribute to the adhesion between cells and a substrate, such as collagen, fibronectin, vitronectin, laminin, nidogen, tenascin, thrombospondin, von Willebrand factor, osteopontin, fibrinogen, elastin, or proteoglycan. The treatment for intensifying the adhesion between a biological material and constructs includes subjecting the surface of the constructs to plasma treatment, hydrofluoric acid treatment, or ultraviolet irradiation treatment, thereby removing the broken pieces of the supports from the surface of the constructs which has been left during separation of the constructs from the substrate, and/or cleaning the surface of the constructs.

In the present invention, handling of a biological material includes various operations such as, but not limited to: transporting the biological material; transferring the biological material to other medium having a different composition from the original one; growing the biological material in a stationary culture at a constant temperature and pH, and in a constant dissolved gas composition; labeling the biological material with a fluorescent substance; observing the biological material in a microscopic field; injecting a substance into or sucking the biological material under a microscope, piercing the biological material with electrodes, or bringing a solid probe into contact with the biological material, thereby measuring the physical properties such as rigidity; and measuring the cell movement, movement of cell organelle, and molecular behavior using optical forceps or any other physical means in a non-contact manner. The transportation of the biological material includes, but not limited to: transporting the construct, to which the biological material has been adhered, by holding it with a solid such as a holding pipette; and transporting the construct with a liquid flow generated by jetting the medium from a pipette.

In the present invention, the interaction between biological materials includes, but not limited to: interaction incident to a generation phenomenon such as cell differentiation or morphogenesis; neurophysiologic interaction between neurons, between a neuron and a muscle cell, or between a neuron and a sensory cell; immunological interaction between an antigen presenting cell and a T-cell, or between an antigen presenting cell and a B cell; interaction between an epithelial cell and a mesenchymal cell, or between a neuron and a glia cell; cell level interaction including interaction between a humoral factor such as a hormone or cytokine and the target cell; interaction on the cell organelle level; interaction on the molecular aggregate level contributing to duplication, transfer, translation, signal communication, respiration, and metabolism; interaction between an enzyme and a substrate and/or a regulatory factor; and the change of association conditions in the three-dimensional structure or aggregation state of a biopolymer.

In the present invention, the analysis of the interaction between biological materials include, but not limited to, the morphological change of cells or cell organelle, gene expression, production of protein or other biomolecule, intracellular ion concentration changes, enzymatic activity, biochemical changes such as the change of association conditions of molecular aggregates or biopolymer, and physical changes such as the changes in cell membrane fluidity and membrane excitability. Among them, morphological change of cells or cell organelle may be observed using an optical microscope. The gene expression or other biochemical change, and some physical changes such as the change in cell membrane fluidity may be observed using an optical microscope with color reaction, fluorescence-labeled technique, or one molecule imaging technique. The changes in the intracellular ion concentration or membrane excitability may be measured using microfabricated electrodes provided on the surface of the constructs to which cells are to be adhered.

The instrument of the present invention for handling a minor amount of a biological material includes constructs and a base holding the constructs in a releasable manner. The base may be an adhesive elastic base. The instrument may be made through: a step of holding constructs, which have been integrated with a substrate via supports, on the surface of the base which holds the constructs in a releasable manner; a step of breaking the supports by a thrust, and holding the constructs on the surface of the base thereby separating them from the substrate. The step of holding the constructs on the surface of the base which holds the constructs in a releasable manner is a step of pressing the base thereby attaching the constructs to the surface of the base, the step of attaching may be followed by a step of breaking the supports by the pressure generated by the thrust, and holding the constructs on the surface of the base thereby separating them from the substrate. In the method of making an instrument, the step of attaching the constructs to the surface of the base and the step of separating the constructs from the substrate are carried out for each construct, and may include a step of pressing a base having one of the constructs with another base having the other construct, thereby forming a three-dimensional construct wherein the surface of one of the constructs is adhered to the other construct.

The substrate is not particularly specified as long as it has a shape or ingredient which allows the formation of the supports and the constructs, but is preferably made of a metal, semiconductor, ceramic, glass, resin, and/or composite thereof. The supports are not particularly specified as long as they have a shape or ingredient allowing the formation and hold for the constructs thereon, but are preferably formed by removing at least a part of the supporting material formed between the substrate and the constructs. When the supports are formed by removing at least a part of the supporting material, the supports for holding the constructs are formed without changing the arrangement of the constructs. Alternatively, the supports may be formed by forming a sacrificial layer having a communicating region communicated with the substrate, forming the constructs so as to fill the communicating region of the sacrificial layer, and then removing the sacrificial layer. In this case, the communicating region works as the supports. When the supports are formed by removing the sacrificial layer, the supports for holding the constructs are formed without changing the arrangement of the constructs.

The shape of the supports is not particularly specified as long as the supports are broken by the external pressure, but is preferably a column. When the supports have a column shape, they hold the constructs, and are broken by the thrust from the top of the constructs.

The instrument of the present invention is made through sterile treatment, or using a material or method nontoxic to living body, according to the biological material to be adhered and the type of the interaction to be analyzed.

The constructs of the present invention are not particularly specified as to their shape or material, and may be natural or artificial constructs. The constructs of the examples herein have a plate, disk, or frustum shape, but may have any shape as long as they allow adhesion of a minor amount of a biological material thereto. For example, the constructs may have a three-dimensional shape such as a tetrahedron, pyramid, or quadrangular pyramid. The constructs may be integral, or composed of a plurality of segments having fixed or movable junctions.

The three-dimensional structure of the constructs of the present invention may be formed through removal of the sacrificial layer using a combination of prior art micromachining techniques such as photolithography and etching (EE Text Sensor Micromachine Kogaku, Hiroyuki Fujita, Ohmsha, Ltd. (2005)). Alternatively, the below-described impression transfer method (described in Japanese Patent Application No. 2007-10867 and Onoe, H. et al, Journal of Micromechanics and Microengineering, 17: 1818-1827 (2007)) may be used. In the examples of the present invention, the impression transfer method is carried out by pressing the base against the constructs adhered to a substrate via supports, thereby separating the constructs from the substrate to be held on base, and transferring the separated constructs onto the base. The operation is repeated several times thereby making constructs having a three-dimensional shape. More specifically, a first construct separated from a first substrate and held on a first base, and a second construct separated from a second substrate and held on a second base are attached to make a composite of the first and second constructs, and the composite is held on the first or second base thereby making a construct having a three-dimensional shape. The operation may be further repeated for a third construct held on a third base, a fourth construct held on a fourth base, and subsequent constructs in the same manner. Alternatively, constructs having a three-dimensional shape may be made using a combination of the prior art micromachining technique and the impression transfer method.

The constructs of the present invention may be a molded article produced using a die having a three-dimensional shape made by the prior art micromachining technique and/or the impression transfer method, which are used alone or in combination. The molded articles may be made of, for example, but not limited to, plastic, ceramic, hydrogel, and composites thereof.

The base of the present invention is not particularly specified as long as it holds a construct in a releasable manner. The base holds the constructs in a releasable manner according to a physical or chemical principle. The method along a physical principle include the release of the constructs from the base upon the application of stress, pressure, vibration, ultrasonic wave, light, electricity, heat, or static electricity. The method along a chemical principle includes the release of the constructs from the base by changing the concentration of ions or other substance, and changing the ionic strength.

The base which holds the constructs in a releasable manner along a physical principle, in particular, the substance which changes in the viscoelasticity, surface hydrophilic, surface tension, surface flatness, surface charging upon the application of stress, pressure, vibration, ultrasonic wave, light, electricity, heat, or static electricity is evident to those skilled in the technical field of the present invention. Preferred examples of the base which holds the constructs of the present invention, and release the constructs upon application of stress, pressure, vibration, or ultrasonic wave include, but not limited to, silicon rubber, polyimide, PET, and PDMS, and other adhesive elastic bases.

The base which holds the constructs in a releasable manner along a chemical principle is also evident to those skilled in the technical field of the present invention. Specificity in molecule discrimination such as, but not limited to, antigen-antibody reaction, enzyme-substrate or enzyme-inhibitor interaction, or interaction between polyhistidine and nickel ions may be used. For example, one of a pair of molecules, which specifically interact with each other, is adhered to a construct, and the other molecule is adhered to a base. As a result of this, the construct is held on the base by the interaction between the molecules, and the construct is released from the base upon the addition of either of the molecules not adhered to the solid phase. Alternatively, an adhesive for bonding the base and the construct, and a reagent for inactivating the adhesive may be used. In some combinations of a base and a construct, the base and the construct are intrinsically sticking to each other. In such cases, the construct may be released from the base by the addition of a substance such as a surfactant for weakening the bond between the base and the construct.

When the base of the present invention is an adhesive elastic base which releases the constructs upon application of stress, the constructs are released from the base without giving any influence on the biological material adhered to the constructs. When the constructs are released from the base along any other physical or chemical principle, those skilled in the technical field of the present invention may select an appropriate method according to the type or properties of the biological material, or the intended use of the constructs to which the biological material is adhered.

The arrangement of the constructs in array on the base allows high-throughput analysis of the interaction of a specific combination of minor amounts of biological materials.

In the base of the present invention, the part holding the constructs may be formed in the form of a projection. When the base of the present invention is made of an elastic material such as silicon rubber, polyimide, PET, or PDMS, at least a part of the projection of the elastic base is locally deformed with the constructs held on the base, thereby indirectly breaking the supports holding the constructs. Furthermore, the constructs are separated from the substrate without deteriorating the accuracy of positions in the arrangement.

The projection is not particularly specified as long as it contacts with at least a part of the constructs to be locally deformed, so as to break the supports. The projection is preferably made of an elastic base which imparts adhesiveness. When the projection is made of an elastic base, at least a part of the projection of the elastic base is locally deformed with the constructs held on the base, thereby indirectly breaking the supports holding the constructs.

The base may have a plurality of projections arranged or formed in such a manner that the projections and the constructs in contact with the projections are off-centered. When a plurality of projections is provided, many constructs are separated at a time. Furthermore, when the projections and the constructs in contact with the projections are arranged or formed off-centered, the respective projections on the base are locally deformed to indirectly break the supports holding the constructs. In addition, the constructs are separated from the substrate with no deterioration of accuracy of positions in the arrangement.

Some projection on the base may have a different height from the other projections. When some projections have a different height from the other projections, the constructs held by the supports on the substrate at a height to be brought into contact with the projections are selectively separate from the substrate.

This allows the control of the arrangement density of the constructs on the base. More specifically, some constructs arranged or formed on the substrate in a high density are selectively separated, thereby arranging the constructs on the base in a low density. The operation may be repeated thereby arranging the constructs more widely in arbitrary positions on the base. The control of the arrangement density is cost-effective particularly when expensive materials are integrated.

In addition, the projection is not particularly specified as to its dimension or shape, and may have any dimension or shape such as an angular, spherical or columnar shape.

In the present invention, the process of breaking the supports for holding the constructs is not particularly specified as long as the supports are broken, but is preferably mechanical destruction such as thrust or shear destruction. The mechanical destruction such as thrust or shear destruction allows indirect destruction of the supports holding the constructs, whereby the constructs are separated from the substrate with no deterioration of the accuracy of position in the arrangement.

In the present invention, the term "optical transparency" refers to that light in the visible light region passes through the constructs. When the biological material adhered to the surface of the constructs is observed under an epifluorescence microscope or a total reflection fluorescence microscope, it is preferred that light in the ultraviolet region pass through the constructs. When an infrared microscope is used, it is preferred that light in the infrared region pass through the constructs. Furthermore, it may be preferred that the constructs of the present invention have a flat surface, uniform optical properties such as the internal refractive index, and optical properties suitable for the application of various optical techniques such as a phase contrast microscope, a differential interference microscopy, or optical forceps.

The self-assembling of the constructs of the present invention is achieved as follows: the constructs randomly collide with each other in a medium to form aggregates of the constructs through physical attraction and/or repulsive force, or chemical or biological bonding, such as, but not limited to, friction force, surface tension, hydrophobic bond, magnetic force, or static electricity, the constructs being arranged according to a specific pattern in the aggregates. The self-assembling of the constructs of the present invention may be achieved by, for example, complementary engagement between a concavity on the surface of one construct and a convexity on the surface of the other construct (for example, see Yeh, H. J. J. and Smith, J. S., Ieee Photonics Technology Letters: 6, 706-708 (1994)), or by the contact between hydrophilic and/or hydrophobic areas provided on the surface of the constructs, wherein the hydrophilic area on the surface of one construct is brought into contact with another hydrophilic area on the surface of the other construct, and/or the hydrophobic area on the surface of one construct is brought into contact with another hydrophobic area on the surface of the other construct (for example, see Bowden, N. et al., Langmuir: 17, 1757-1765 (2001)). Alternatively, the self-assembling may be achieved by chemical or biological bonding based on molecular recognition specificity, such as, but not limited to, reaction between an antigen and an antibody, interaction between an enzyme and a substrate or inhibitor, or interaction between polyhistidine and nickel ions. Alternatively, the self-assembling may be achieved by a combination of any of the above ones.

When the constructs are in the form of a plate or a disk, they may not form aggregates during three-dimensional free movement in a medium. In such cases, a liquid which is insoluble in the medium is added to the medium, and the constructs are floated at the interface between the medium and the insoluble liquid, so as to allow only two-dimensional free movement of the constructs, thereby promoting the formation of aggregates. The liquid insoluble in the medium is preferably a liquid having a greater specific gravity than the medium, or a liquid whose surface tension between the liquid and the base is smaller than the surface tension between the medium and the base.

In order to bond a specific combination of biological materials to each aggregate, the number of the self-assembling constructs composing the aggregates and the arrangement pattern of the constructs may be appropriately controlled. For example, two types of constructs S1 and S2 are designed so that they aggregate one by one, and an instrument T1 consisting solely of a construct S1, an instrument T2 consisting solely of a construct S2, and different types of cells C1 and C2 are prepared. Subsequently, the instrument T1 is exposed to an isolated suspension of the cell C1, and the instrument T2 is exposed to an isolated suspension of the cell C2. Thereafter, the constructs S1 and S2 are released from the T1 and T2, and mixed in a medium, thereby forming aggregates each composed of one each of the constructs S1 and S2. At this time, interaction between one each of the cells C1 and C2 occurs on the surface of the aggregates. The surface of the constructs S1 and S2 may have been microfabricated so as to allow the adhesion of the cells C1 and C2 in a specific direction. For example, when the cell C1 is a neuron and the cell C2 is a muscle cell, the constructs S1 and S2 forms an aggregate such that the muscle cell is arranged to be adjacent to the axon of the neuron. The use of the aggregates allows concurrent real time analysis of many samples each including synapse formation between a single neuron and a single muscle cell.

In the instrument of the present invention composed of constructs and a base for handling a minor amount of a biological material, the solvent in which the base holds the constructs may be an aqueous, oil, or amphipathic solvent. When the biological material is a cell, the solvent is preferably an aqueous solution such as a culture solution suitable for the cell.

The specific interaction between a part of the surface of the base and another part of the surface of the constructs include, but not limited to, hydrophobic interaction, electromagnetic interaction, interaction between a biomolecule and a specific binding partner for the biomolecule, and interaction between a metal ion and a specific chelating agent for the metal ion. The hydrophobic interaction may be caused by the direct contact between a part of the surface of the base and another part of the surface of the constructs, the surfaces having been treated to become hydrophobic. Alternatively, the interaction may be mediated by a hydrophobic liquid such as oil droplets. The hydrophobic treatment may be achieved by, for example, bonding a hydrophobic molecule such as alkanethiol to the surfaces of the constructs and base using a well-known surface finishing technique such as a self-assembling method. The electromagnetic interaction includes, but not limited to, interaction using electrostatic attraction or surface charge attraction, interaction using a magnetic field between magnets, and interaction using an electric field and an electric dipole moment induced thereby. The electromagnetic interaction using an electric field and an electric dipole moment induced thereby includes, but not limited to, dielectrophoresis and electrostatic orientation. The interaction between a biomolecule and a specific binding partner for the biomolecule includes, but not limited to, interaction between a saccharide and lectin, interaction between antigens, and interaction by complementary pairing of nucleic acids. Alternatively, the constructs may be held on the base by the combination of the interaction between the constructs and the base which engage with each other in a specific direction by having a form engageable with each other, and the interaction between the constructs and the base caused by blocking of openings in the base by the constructs under hydrostatic pressure. Alternatively, the constructs may be held on the base by a combination of two or more of the above interactions.

In the instrument of the present invention composed of constructs and a base for handling a minor amount of a biological material, at least two constructs and the base holding the constructs in a releasable manner may be made by, for example, coating the surface of the base with a layer of a substance such as gelatin which is readily removed by a minor force, and subjecting the coating to well-known micromachining so as to allow mounting of the constructs. The minor force refers to the force generated by the minor amount of a biological material. When the minor amount of a biological material is a cell, in order to allow the base to hold the constructs in a releasable manner, the base keeps the constructs at a predetermined position so as not to move the constructs when the instrument is moved or a solution is poured onto the base, wherein the constructs are readily released from the base when the constructs are drawn by a force generated by the cell movement.

The instrument of the present invention including at least two constructs and a base holding the constructs in a releasable manner may be used as a three-dimensional scaffold of cells, which is cultured with the cells kept in a specific three-dimensional orientation, or used for the formation of aggregates wherein the constructs cover one or more cells so as to substantially form a convex polyhedron. As a result of this, the cells are culture under conditions similar to their biological tissue. Therefore, the instrument is useful for creation of cell drugs for regenerative medicine.

The constructs used for forming the aggregates of the present invention composed of cells and constructs may be driven individually or two or more of them concurrently, depending on whether the aggregates include an actuator or connected to an external power supply. Alternatively, when the constructs contain a cell such as a heart muscle cell which causes morphological change, the constructs may be driven by cell movement. The actuator may be made using well-known micromachining technique or MEMS technique. The aggregates of the present invention including cells and constructs may be used for measuring the physical properties of the surface of one or more cells. The physical properties refer to, but not limited to, elasticity, hardness, surface potential, and membrane transportation. The physical properties related to the membrane transportation include osmotic pressure, active transportation, and other properties related to mass transfer by biological membranes. When the physical properties of the cell surface is measured, the constructs preferably include a physical property sensor such as a sensor for detecting a mechanical rate such as displacement, speed, force, or pressure, or an ion sensor including ion-selective electrodes or ion-sensitive electric field transistor (ISFET). The physical property sensor may be made using well-known micromachining technique (see EE Text Sensor Micromachine Kogaku, Hiroyuki Fujita, Ohmsha, Ltd. (2005)). The constructs and/or the base may include electrodes on a printed wiring board to be bought into contact with cells. Upon engagement of the constructs with the base, the electrodes are connected to an external power supply with the purpose of giving electric stimulation or measuring the electric potential. Alternatively, through holes, which are pores penetrating the constructs and/or the base and have a smaller diameter than one cell, may be provided so as to be electrically sealed by the cell membrane, for the purpose of carrying out electrophysiological measurement by a so-called auto-patch method. In this case, the through holes may be arranged such that the through holes in the constructs communicate with the through holes in the base upon engagement of the constructs with the base. The printed wiring board and through holes may be made by well-known micromachining technique or MEMS technique.

Examples of the present invention are described below. However, these examples are exemplary embodiments, and will not limit the scope of the present invention.

### Example 1

### Cell culture

NIH3T3 cells of a mouse fibroblast line and the HepG2 cells of a human liver cancer cell line were used as the cells having adhesiveness to substrates. These cells were cultured in a Dulbecco's Modified Eagle's Medium (DMEM, 11885, Gibco) mixed with 10% fetal bovine serum (FBS, F7524, SIGMA) and 5% L-glutamine-penicillin-streptomycin solution (G1146, SIGMA) at 37°C in a 5% CO₂ atmosphere. When the cells became confluent, they were dissociated using a 0.25% trypsin-EDTA solution (T4049, SIGMA), and subcultured at a ratio of 1:5. The cells were fluorescence-labeled using Fluorescence Cell Linker Kit (MINI26-1KT PKH26 red fluorescence linker and MINI67-1 KT PKH green fluorescence linker) in accordance with the manufacturer's protocol. The fluorescence microscope observation used a differential interference microscope equipped with a fluorescent filter (double band filter U=M51003D/TR, Olympus Corporation).

### Cell adhesiveness to substrate

When cells are brought into contact with the instrument of the present invention, it is preferred that the cells selectively adhere to the surface of the constructs in preference to the surface of the elastic base. In view of this, the 3T3 and HepG2 cells were examined for their adhesiveness to the surface of thermally grown silicon dioxide, which is the material of the constructs of the example, and to the surface of poly-dimethylsiloxane (PDMS, Sylgard184, Dow Corning), which is the material of the elastic base of the example. They were tested after being subjected to oxygen plasma treatment (Compact Etcher FA-1, Samco International, Inc., 50 W, 10 mL/s oxygen, one minute), collagen treatment (immersion for three hours in 5% collagen 1-P, Cellmatrix, type 1-P, Nitta Gelatin Inc., dissolved in 1 mM hydrochloric acid, Kanto Chemical Co., Inc.), or untreated.

As shown in Table 1, the 3T3 and HepG2 cells adhered to the surface of collagen-treated silicon dioxide, but never adhered to the surface of untreated PDMS. Therefore, in the following example, the surface of the constructs to be adhered to the cells was made of collagen-treated silicon dioxide, and the elastic base was made of untreated PDMS.

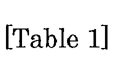

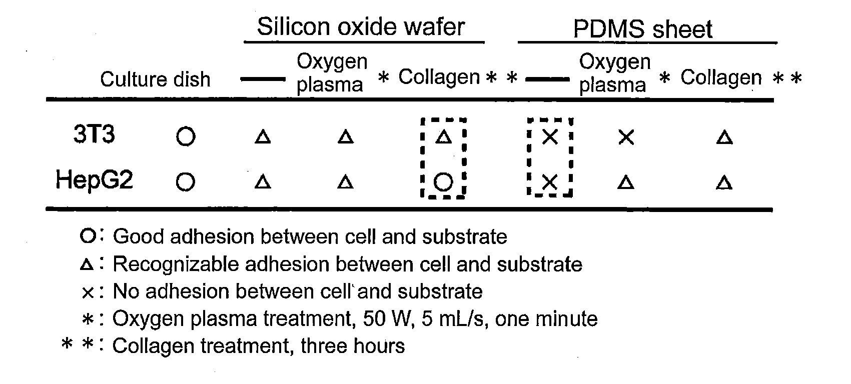

### Example 2

### Processing of the instrument of the present invention for handling a minor amount of a biological material

The instrument of the present invention was made as follows. The constructs made of SiO₂ were produced by a micromachining technique, and fixed on a base made of PDMS. The impression transfer method used for the micromachining of the constructs of the present invention is described in detail in Japanese Patent Application No. 2007-10867 and Onoe, H. et al., Journal of Micromechanics and Microengineering, 17; 1818-1827 (2007). In brief, the starting material was silicon on insulator (SOI) wafer (device silicon layer thickness: 3 µm, embedded oxide (SiO₂) layer thickness 2 µm, substrate silicon layer thickness 450 µm, Shin-Etsu Chemical Co., Ltd.). The embedded oxide layer was used as the material of the constructs. The device silicon layer was etched by the inductively coupled plasma-reactive ion etching method (ICP-RIE, Multiplexer, STS) using S1805 photo resist (Shipley) as the masking layer. After removing the photo resist layer, the embedded oxide layer was etched by hydrofluoric acid (HF, 50%, STELLA Chemifa) to form the shape of the constructs (Fig. 2(B)). The constructs of the example were designed so as to have a diameter of 50 to 75 µm.

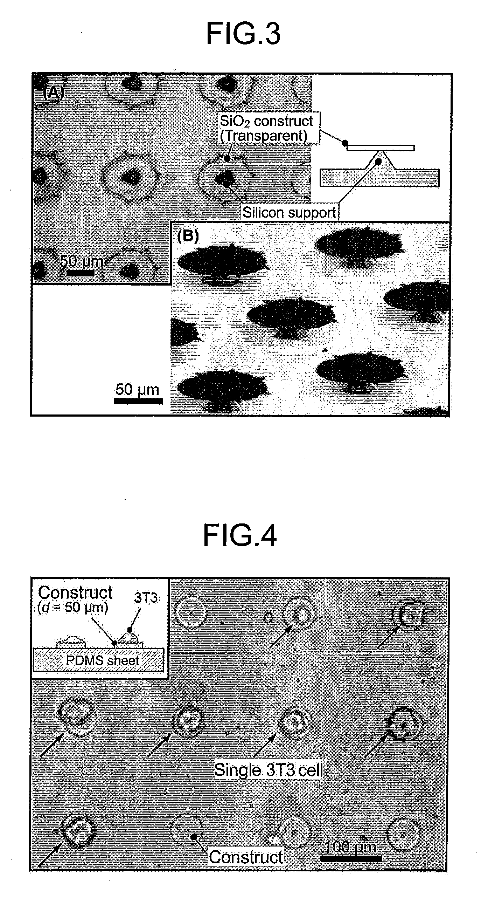

The part of the device silicon layer remaining on the SiO₂ layer was removed by the ICP-RIE method, and the substrate silicon layer below the SiO₂ layer was anisotropically etched with tetramethylammonium hydroxide (TMAH, 22%, Kanto Chemical Co., Inc.) at 80°C. The anisotropy etching reaction was stopped immediately before the SiO₂ layer was completely separated from the substrate silicon layer. In this manner, a pyramid-like silicon structure holding the constructs was formed (Fig. 2(C)). As shown in Fig. 3, the constructs of the present example were held at the peak of the pyramid-like silicon structure in a narrow area of several nanometers. Therefore, the constructs were easily separated from the substrate silicon layer on the wafer by breaking the supports at the peak. Before separating the constructs, in order to intensify the adhesion between the cells and constructs, the constructs on the wafer were immersed for three hours in a collagen solution in hydrochloric acid described in Example 1.

Thereafter, the constructs were separated from the SOI wafer by the impression transfer method, and held on the PDMS elastic base by adhesion. A PDMS sheet of 1 mm thickness was placed above the constructs on the wafer (Fig. 2(D)), and then removed (Fig. 2(E)). The outcome was the instrument of the present invention for handling a minor amount of a biological material including the constructs held on the surface of the elastic base with a part of the construct exposed at the outer surface. The efficiency of transfer of the constructs to the elastic base was high, and about 90% of the constructs were held on the surface of the elastic base with the arrangement pattern on the wafer maintained (Fig. 2(F)).

### Example 3

### Constructs to which cells are adhered

In order to confirm the selective adhesion between the cells and constructs, the culture solution of Example 1 containing the suspended 3T3 cells at a concentration of about 1 x 10⁵/mL was placed on the surface of the instrument of Example 2. The constructs included in the instrument of the example had a diameter of 50 µm, which was almost the same as the dimension of one extended 3T3 cell.

Fig. 4 is a micrograph of 3T3 cells cultured for four days on the surface of the constructs held on the PDMS elastic base in array. As shown in Fig. 4, the 3T3 cells adhere only to the SiO₂ constructs, but not to the elastic PDMS base. The result indicates that the selective adhesion of the cells to the SiO₂ constructs coated with collagen was successively achieved. The cells adhered to about 50% of the all constructs.

### Example 4

### Treatment of constructs to which cells are adhered

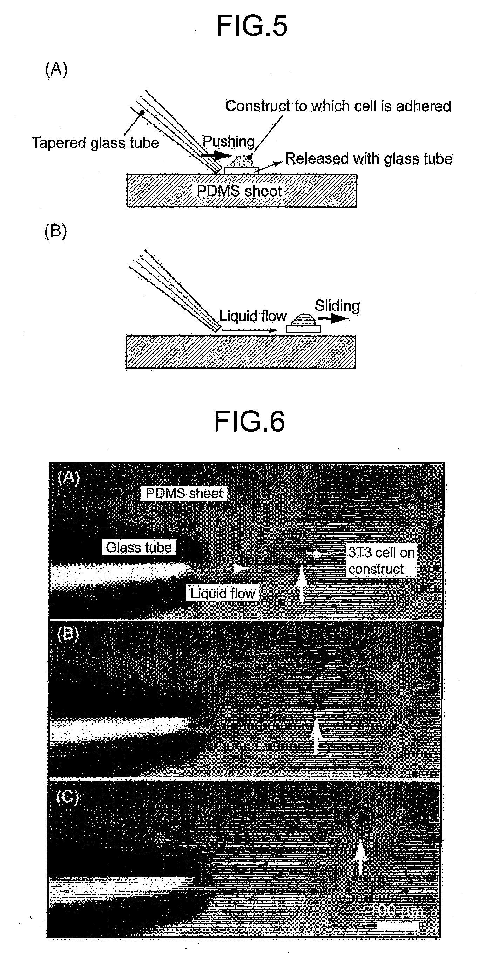

The constructs to which the cells are adhered were physically released from the elastic base after cell culture. A pulled glass tube (tip diameter: about 150 µm) was mounted on a micromanipulator, and, as shown in Fig. 5(A), the construct was pushed in a horizontal direction to be released from the base. Subsequently, the culture solution was ejected from the glass tube such that its flow moves the construct on the elastic base.

The sequential photographs of Fig. 6 show that a construct to which a 3T3 cell is adhered, which had been released from the PDMS elastic base, was slid on the elastic base by a liquid flow from a glass tube. The construct released from the elastic base never adhered to the elastic base. The liquid flow from the glass tube was strong enough for moving the construct, but not enough for tearing the cell from the construct. Therefore, the liquid flow moved the construct with the cell adhered thereto. The construct was released from and moved on the elastic base, and then stained with trypan blue (MP Biochemicals, Inc.), but the dye did not enter into the cell. The fact indicates that the cell adhered to the construct was alive after the release and moving of the construct. The cell adhered to the construct was handled in the same manner as a floating cell using a liquid flow. When no liquid flow was blown on the construct, the construct to which the cell is adhered remained in the same position. The fact indicates that the present invention has allowed free handling of a cell adhered to a substrate.

### Example 5

### Treatment of cells of different cell types

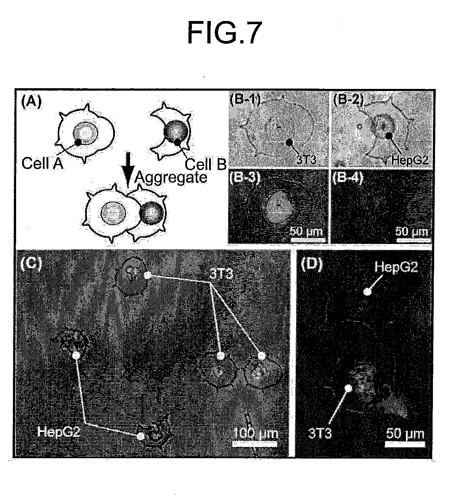

Treatment of cells of different cell types by the instrument and method of the present invention is described below. Fig. 7(A) is a schematic diagram of self-assembling of constructs achieved by complementary engagement between a concavity on the surface of one construct and a convexity on the surface of the other construct. In the present example, a 3T3 cell was adhered onto a construct having a convexity, and a HepG2 cell was adhered to another construct having a concavity. The constructs of the example had a diameter of 75 µm without the convexity or concavity, and had spines around them thereby preventing nonspecific aggregation. The construct having a convexity and the construct having a concavity held on separate elastic bases to make instruments. The suspensions of the 3T3 and HepG2 cells were placed on the respective instruments, and cultured for four days (Figs. 7(B-1) to 7(B-4)). Thereafter, the constructs were released from the elastic bases, and a liquid flow from a glass tube was blown on the constructs, thereby moving the constructs from the respective instruments to one culture dish having a diameter of 5 cm (2 inch).

Figs. 7(C) and 7(D) show the coexistence of two different constructs on a culture dish. In Fig. 7(C), the distance between the construct to which the 3T3 cell is adhered and the construct to which the HepG2 cell is adhered was about 500 µm. In Fig. 7(D), the distance between the construct to which the 3T3 cell is adhered and the construct to which the HepG2 cell is adhered was about 100 µm. These results indicate that the instrument and method of the present invention allows treatment of cells of different cell types on the level of a single cell or several cells.

### Example 6

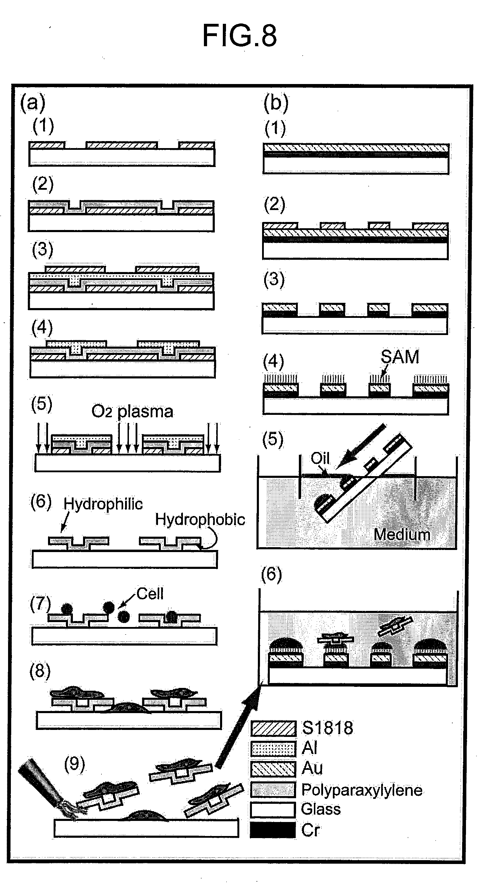

Fig. 8 shows schematic views illustrating a method for making the instrument of the present invention for handling a minor amount of another biological material. Fig. 8(a) illustrates the steps for making a glass substrate having thereon a construct of the present invention made of polyparaxylylene. A sacrificial layer made of S1818 photo resist (Shipley) (approx. 3 µm thick) was formed on a glass base (a1), in such a manner that the areas to come into contact with the construct were exposed. The contacting areas were narrow enough for allowing easy release of the construct from the glass substrate. Subsequently, a polyparaxylylene layer (approx. 5 µm thick) was formed on the sacrificial layer using a Labcoater PDS 2010 system (Shipley) (a2). Furthermore, aluminum (a3) and S1818 (a4) were deposited thereon, and O₂ plasma etching was carried out (a5) at 25 W and an oxygen flow rate of 10 ml/s for 18 minutes. The parts of the polyparaxylylene and S1818 layers which had not been masked by aluminum were completely removed. Thereafter, the aluminum layer was removed. The exposed surface of the polyparaxylylene layer, which had been hydrophobic in that state, was subjected to O₂ plasma treatment again; thereby selectively make the surface treated with O₂ plasma (a6) hydrophilic. The surface of the polyparaxylylene layer facing the glass substrate and the surface in contact with the glass substrate remained hydrophobic because they were not exposed to O₂ plasma. Cells were inoculated on the construct (approx. 50 µm in diameter) thus made (a7), and the cells adhered to the hydrophilic parts of the surface of the construct (a8).

Fig. 8(b) shows the procedure for making the base holding the constructs by hydrophobic interaction. Chromium and gold were vapor deposited on a glass substrate (b1), and the chromium and gold layers were etched using S1818 (b2) such that the chromium and gold layers had the same dimension as the constructs (diameter: about 50 µm) (b3). The gold layer was subjected to O₂ plasma treatment for 10 seconds at 25 W and an oxygen flow rate of 10 ml/s, rinsed with ethanol, and dried in a nitrogen atmosphere thereby forming a uniform self-assembling monomolecular layer (SAM) on the gold surface. Thereafter, the gold surface was immersed in an ethanol solution containing 1 mM 1-octadecanethiol (Aldrich) for 30 minutes, and rinsed with ethanol several times thereby making the gold surface (b4) hydrophobic. The liquid surface in the container filled with the cell culture solution was surrounded with a silicone rubber sheet, and squalene was dispensed in layers over the surrounded surface. The base shown in Fig. 8 (b4), which has been treated to become hydrophobic, was sunk slowly in the culture solution through the liquid surface covered with squalene (about 1 cm per second); squalene oil droplets were captured only on the gold surface (b5), which has been hydrophobic. As shown in Fig. 8 (a9), the constructs to which the cells are adhered were released from the glass substrate by a water flow generated by pipetting, and moved to the culture solution containing the base which captured squalene; the hydrophobic surface of the constructs contacts with and adhere to the squalene oil droplets (b6). In this manner, the constructs to which the cells are adhered were mounted on the base according to an intended arrangement pattern. The constructs mounted via the squalene oil droplets were stably held on the base.

### Example 7

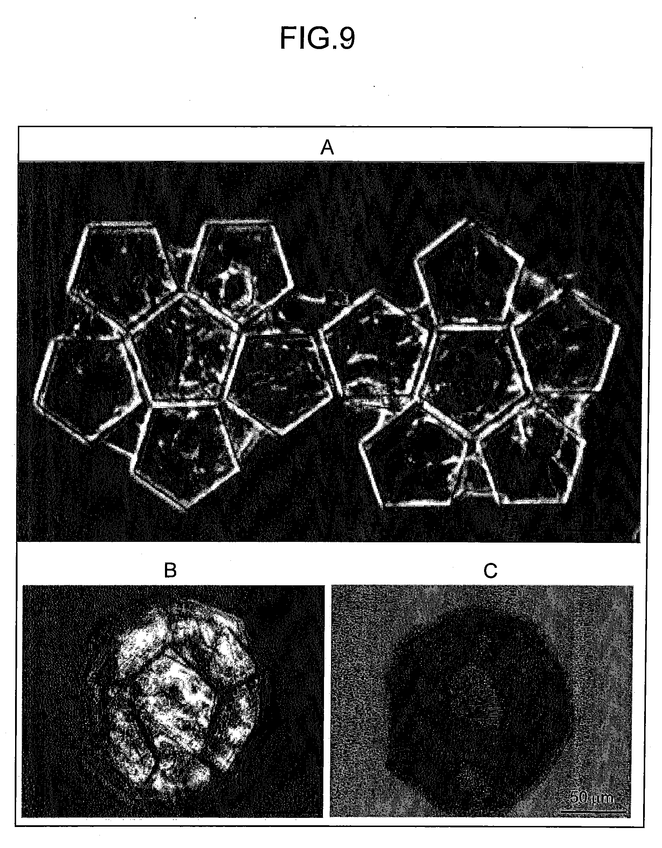

Fig. 9 is a micrograph showing the experimental result of the example of aggregates composed of cells and constructs using the instrument of the present invention for handling a minor amount of a biological material. The instrument used in the present invention was made by the following procedure. A glass substrate was spin-coated with a 2% gelatin aqueous solution at 200 rpm, further coated with a polyparaxylylene layer, and subjected to O₂ plasma treatment through an aluminum mask. After removing the unmasked parts of polyparaxylylene and gelatin layers, the substrate was spin-coated with a hydrophilic phospholipid polymer (2-methacryloyloxyethyl phosphorylcholine polymer, hereinafter referred to as "MPC polymer"), the aluminum mask was removed using tetramethylammonium hydrooxide (NMD-3, Tokyo Ohka Kogyo Co., Ltd.), and the MPC polymer was removed from the aluminum layer by a lift-off method. The polyparaxylylene layer was in an equilateral pentagon form, and arranged in proximity to each other. Fig. 9-A is a micrograph of the constructs one day after inoculating the 3T3 cells thereon. The constructs were evenly developed on the base. When the base was prodded with a glass tube, as shown in Figs. 9-B (phase contrast microscope photograph) and 9-C (bright field micrograph), the constructs were drawn by the cells to rise, and formed aggregates surrounding the cells. In this manner, through the use of the instrument of the present invention for handling a minor amount of a biological material, aggregates in the form of a convex polyhedron, wherein cells are surrounded by artificial constructs, are formed. Alternatively, a nest of aggregates of different sizes may be formed.

### Example 8

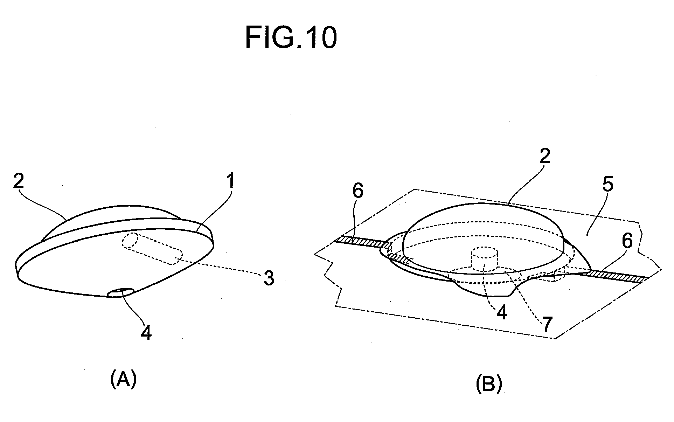

Fig. 10 shows another example of the instrument of the present invention for handling a minor amount of a biological material. In the construct shown in Fig. 10(A), a very small cylindrical magnet 4 is embedded in a construct 1 having thereon a cell 2, and a through hole 4 is formed in the construct 1. In Fig. 10(B), a construct 1 having thereon a cell 2 is engaged with a base 5. A conductor wiring 6 is printed on the surface of the base 5, through which a voltage is applied to a part of the cell to give stimulation. In Fig. 10(B), after the construct 1 is engaged with the base 5, the cell 2 spreads pseudopodia to the surface of the base 5, and a part of them is in contact with printed wiring 6. The base 5 has a through bore 7 in liquid communication with a through bore 4 in the construct 1. This allows electrophysiological measurement of the cell 2 adhered to the construct 1 by the patch clamp method. The arrangement and/or orientation of the construct 1 on the base 5 may be changed using a very small magnet 4. In Fig. 10(B), the construct 1 includes no printed wiring. However, according to the present invention, printed wiring may be formed on the surface of the construct, the printed wiring on the base and construct electrically connecting to each other upon engagement between the base and construct. The printed wiring on the surface of the construct may be arranged in such a manner that it contacts with a specific region of a cell on the surface to be adhered to the cell. The printed wiring may be formed by a combination of treatment for only a part of the surface so as to allow cell adhesion thereto, and/or treatment for other part of the surface so as to impede cell adhesion thereto, for example, treatment to make hydrophobic. The example shown in Fig. 10 includes a magnet, a through hole, and printed wiring. In addition, the constructs and/or base including one or more of these components and a sensor or actuator are also included in the present invention.

## Claims

1. An instrument for handling a minor amount of a biological material, comprising a construct and a base, wherein:
the base holds the construct in a releasable manner;
the construct is held on a surface of the base in such a manner that a part of the construct is exposed externally on the outer face of the instrument; and
at least a part of a surface of the constructs is exposed externally on the outer face of the instrument being fabricated so as to allow the adhesion of the minor amount of the biological material thereto.

2. The instrument of claim 1, wherein:
the base is made of adhesive and elastic material;
the construct is held on the surface of the elastic base by adhesion in such a manner that a part of the construct is exposed externally on the outer face of the instrument; and
the construct is released from the elastic base upon application of stress on the elastic base.

3. The instrument of claim 1 or 2, wherein the minor amount of the biological material is a single cell for a single construct.

4. The instrument of claim 3, wherein the biological material is an animal cell, and the surface of the construct exposed externally on the outer face of the instrument has a dimension allowing the extension of only a single animal cell.

5. The instrument of claim 1 or 2, wherein:
the minor amount of the biological material is a single animal cell for two or more constructs;
the surface of a single construct exposed externally on the outer face of the instrument is smaller than the dimension of the single extended animal cell; and
the single animal cell draws the two or more constructs to form a three-dimensional cell scaffold.

6. The instrument of any one of claims 1 to 5, wherein the construct is optically transparent.

7. The instrument of any one of claims 1 to 6, wherein the construct has a self-assembling function.

8. The instrument of claim 7, wherein:
a construct is provided with a concave surface and/or a convex surface;
the self-assembling function is achieved by complementary engagement between the concave surface of one construct and the convex surface of another construct.

9. The instrument of claim 7 or 8, wherein:
a construct is provided with a hydrophilic surface and/or a hydrophobic surface;
the self-assembling function is achieved by a contact between the hydrophilic surface of one construct and the hydrophilic surface of another construct, and/or, a contact between the hydrophobic surfaces of one construct and the hydrophobic surfaces of another construct.

10. A kit for analyzing a minor amount of a biological material, comprising the biological material and the instrument of any one of claims 1 to 9.

11. The kit of claim 10, wherein the minor amount of the biological material is adhered to the instrument.

12. The kit of claim 10 or 11, wherein the biological material is a cell.

13. A method for handling a minor amount of a biological material, comprising:
a step of holding a construct on a surface of a base in a releasable manner, the construct being integrally molded with a substrate and a support which connects the construct to the substrate;
a step of disposing liquid on the construct on the base, the liquid suspending a biological material, so that the minor amount of the biological material is adhered on at least a part of a surface of the construct;
a step of releasing the construct, to which the biological material is adhered, from the base; and
a step of moving the construct to which the biological material is adhered.

14. The method of claim 13, wherein:
the step of holding the constructs on the surface of the base in a releasable manner is a step of attaching the construct on the surface of the base by pressing the construct to the surface of the base; and, following the step of attaching the construct,
the method is further comprised of:
a step of breaking the support by the force of pressing the construct;
separating the construct from the substrate; and
holding the construct on the surface of the base.

15. The method of claim 14, wherein:
the step of attaching the construct to the surface of the base and the step of separating the construct from the substrate are independently carried out for each of a plurality of constructs; and
the method further comprises a step of pressing a base holding one of the plurality of constructs with a base holding another of the plurality of constructs, thereby forming a three-dimensional construct in which one of the plurality of constructs is adhered to the surface of another of the plurality of constructs.

16. The method of claim 14 or 15, wherein:
the base is adhesive and elastic;
the construct is integrally molded with a substrate and a support which connects the construct to the substrate;
the step of attaching the construct on the surface of the base is a step of pressing the adhesive and elastic base to the construct, thereby attaching the constructs to the surface of the elastic base using the elastic force generated in the elastic base;
the step of separating the constructs from the substrate is a step of breaking the supports by the force of pressing the construct, and holding the constructs on the surface of the elastic base by adhesion thereby separating the construct from the substrate; and
the step of releasing the construct, to which the biological material is adhered, from the base is a step of deforming the elastic base under stress thereby releasing the construct, to which the biological material is adhered, from the elastic base.

17. The method of any one of claims 14 to 16, wherein
the method is comprised of, preceding the step of holding,
a step of intensifying the adhesion between the biological material and the construct.

18. The method of any one of claims 13 to 17, wherein the minor amount of the biological material is one cell for one construct.

19. The method of any one of claims 13 to 18, wherein the minor amount of the biological material is a single cell for two or more constructs.

20. The method of any one of claims 13 to 19, wherein the construct has a self-assembling function.

21. A method for analyzing interaction between minor amounts of biological materials, comprising:
a step of preparing two or more instruments of any one of claims 7 to 9, wherein the construct comprising the instruments has been designed such that a construct derived from one of the instruments self-assemble with a construct derived from another of the instruments, but a construct derived from one of the instruments does not self-assemble with a construct derived from the same instrument;
a step of adhering a minor amount of a biological material to the surface of the construct exposed externally on the outer face of the two or more instruments;
a step of releasing the constructs, to which the minor amount of the biological material is adhered, from the base of each instruments;
a step of allowing self-assembling of the constructs, to which the minor amount of the biological material is adhered, in a mixed state, thereby forming an aggregate composed of the constructs derived from different instruments;
a step of allowing reaction to cause interaction between the minor amounts of the biological material adhered to the surface of the constructs forming the aggregate; and
a step of analyzing the interaction between the minor amounts of the biological material for the individual aggregate.

22. The method of claim 21, wherein, the type of the biological materials adhered to the construct is different among the instruments.
